# EUROPEAN PATENT SPECIFICATION

(11) **EP 2 231 091 B1**
(45) Date of publication and mention of the grant of the patent: **24.10.2012**
(21) Application number: 08869646.3
(22) Date of filing: 17.12.2008
(51) Int. Cl.: A61F 13/15, A61F 13/515, A61F 13/511

(54) **ABSORBER AND DISPOSABLE ABSORBENT ARTICLE**
ABSORBER UND SAUGFÄHIGER EINWEGARTIKEL
ELÉMENT ABSORBANT ET ÉLÉMENT ABSORBANT JETABLE

(30) Priority: 08.01.2008 JP 2008000927
(43) Date of publication of application: 29.09.2010
(73) Proprietor: Livedo Corporation, Shikoku-Chuo-shi, EHIME 799-0122 (JP)
(72) Inventor: FUJIOKA, Masaru, Mima-gun Tokushima 779-4104 (JP)
(74) Representative: Müller-Boré & Partner Patentanwälte
(86) International application number: PCT/JP2008/003800
(87) International publication number: WO 2009/087719

(56) References cited:
- EP-A- 0 248 584
- EP-A- 1 016 393
- GB-A- 2 296 445
- US-A- 3 067 747
- US-A- 4 623 340
- US-A- 5 478 335
- US-B1- 6 443 934

## Description

### Technical Field

The present invention relates to an absorber provided in a disposable absorbent article and such disposable absorbent article.

### Background Art

An absorber for use in disposable absorbent articles such as disposable diapers and disposable garments commonly includes an absorber made of pulp fibers (fluffed broken pulp), thermally-bonded fibers, super absorbent polymer powder or the like, and a liquid-permeable sheet member for covering the absorber.

The inventor of the present invention and others have made various kinds of inventions for the purpose of preventing deformation of the absorber due to twisting and improving the absorbing speed (cf. Patent Citations 1 to 3). Deformation such as bunching of the absorber due to twisting will cause disadvantages such as degraded performance in absorbing urine or other bodily fluids and a poor fit to a wearer.
Patent Citation 1: Japanese Patent Laying-Open No. 9-266929
Patent Citation 2: Japanese Patent Laying-Open No. 2007-202575
Patent Citation 3: Japanese Patent Laying-Open No. 2007-215688

EP 0 248 584 A2 discloses a sanitary napkin comprising a liquid-permeable surface material, a liquid impermeable leakproof material and an absorber inserted between said surface material and said leakproof material, said absorber comprising a first absorbent paper, a second absorbent paper and an absorbent material inserted between both papers, said first absorbent paper covering at least a part of said second absorbent paper, said first absorbent paper being fixed at the portion thereof covering the second absorbent paper onto said leakproof material.

US 5 478 335 A discloses an absorbent device having low fluid wet-back and good surface cleanliness and resistance to staining is provided which comprises an absorbent core, a cover sheet on a body facing side thereof and an intermediate layer between the cover sheet and the core wherein the cover sheet comprises an apertured polymer film, for example a flexible polymer net, and the intermediate layer comprises a planar apertured polymer film, for example a flexible polymer net.

US 3 067 747 A discloses a cellulosic product with a top sheet comprising a permeable non-woven web of bonded synthetic hydrophobic fibers, wherein directly beneath the top sheet is a highly absorbent material which may consist of a plurality of absorbent creped cellulose wadding sheets, and wherein beneath the absorbent material is a gauze web. The top sheet partially encircles the absorbent material in face-to-face engagement therewith, marginal portions of the top sheet being folded about the absorbent material sufficiently to overlap marginal portions of the gauze with the overlapped portions being bonded together, such as by being heat-sealed one to the other, to maintain the elements in unitary assembly.

US 4 623 340 A discloses an absorbent product wherein a cover is provided having a pattern of depressed and undepressed areas imposed on its exterior surface, which pattern presents a highly visible color contrast between the depressed and undepressed areas. The cover comprises a relatively light colored, relatively opaque outer cover having an interior layer of relatively dark color placed in face-to-face relationship with the interior surface of said outer cover and co-embossed.

GB 2 296 445 A discloses an absorbent article comprising an absorbent member disposed between a liquid permeable topsheet and an impermeable backsheet and lateral flaps extending from opposite sides, the flaps being folded to form anti-leakage walls and, outwardly of such walls, anti-leakage surfaces. The flaps may be formed by fixing together extended portions of the topsheet and backsheet, by the backsheet or the topsheet alone or by an additional, liquid impermeable sheet and are secured to the absorbent body towards the front and rear thereof.

US 6 443 934 B1 discloses a disposable absorbent article, e.g. a pantiliner pad, for use with a thong shaped undergarment, wherein the pad is of rectangular shape and includes a tapering, e.g. truncated triangle, absorbent core adhesively secured between a rectangular moisture pervious top sheet and a rectangular moisture impervious outer cover. Portions of the top sheet and outer cover extending beyond the margins of the core form a pair of triangular mounting flaps.

### Disclosure of Invention

An object of the present invention is to provide an absorber and a disposable absorbent article which prevent deformation of the absorber due to twisting and has improved absorbing speed.

An absorber according to a first aspect of the present invention provided in a disposable absorbent article includes an assembly at least having assembled fibers, and an upper sheet member made of a liquid-permeable nonwoven fabric. The upper sheet member covers an upper surface and left and right edges of the assembly, and also covers regions at least in the vicinity of the left and right edges on a lower surface of the assembly. The upper sheet member is adhered to the assembly at least in the vicinity of the left and right edges on the lower surface of the assembly.

According to the absorber of the first aspect, the upper sheet member covers the upper surface and left and right edges of the assembly, and also covers regions at least in the vicinity of the left and right edges on the.lower surface of the assembly. The upper sheet member is made of a nonwoven fabric having high liquid permeability, resisting tearing, and having excellent flexibility. Accordingly, the absorber resists tearing and has flexibility. Also, the absorber has improved absorbing speed, so that bodily fluids such as urine pass immediately through the upper sheet member to reach the assembly to be absorbed therein.

Again, the upper sheet member covers not only the upper surface and left and right edges of the assembly, but also regions at least in the vicinity of the left and right edges on the lower surface of the assembly. Further because the upper sheet member is adhered to the assembly at least in the vicinity of the left and right edges on the lower surface of the assembly, the assembly and upper sheet member can be prevented from being separated from each other even when the absorber is twisted, which prevents deformation of the absorber.

An absorber according to a second aspect of the present invention provided in a disposable absorbent article includes an assembly at least having assembled fibers, a lower sheet member adhered to a lower surface of the assembly, and an upper sheet member made of a nonwoven fabric. The upper sheet member covers an upper surface and left and right edges of the assembly, and also covers, from the side of the lower surface of the lower sheet member, regions at least in the vicinity of the left and right edges on the lower surface of the assembly. The upper sheet member is adhered to the assembly with the lower sheet member interposed therebetween, at least in the vicinity of the left and right edges on the lower surface of the assembly.

According to the second aspect, the upper sheet member covers the upper surface and left and right edges of the assembly, and also covers, from the side of the lower surface of the lower sheet member, regions at least in the vicinity of the left and right edges on the lower surface of the assembly. The upper sheet member is made of a nonwoven fabric having high liquid permeability, resisting tearing, and having excellent flexibility. Accordingly, the absorber resists tearing and has flexibility. Also, the absorber has improved absorbing speed, so that bodily fluids such as urine pass immediately through the upper sheet member to reach the assembly to be absorbed therein.

Again, the upper sheet member covers not only the upper surface and left and right edges of the assembly, but also regions at least in the vicinity of the left and right edges on the lower surface of the assembly, from the side of the lower surface of the lower sheet member. Further because the upper sheet member is adhered to the assembly with the lower sheet member interposed therebetween, at least in the vicinity of the left and right edges on the lower surface of the assembly, the assembly and upper sheet member can be prevented from being separated from each other even when the absorber is twisted, which prevents deformation of the absorber.

For the lower sheet member, a material different from the upper sheet member may be selected (e.g., a nonwoven fabric thinner and stronger than the upper sheet member, or a crepe paper which diffuses liquid well). Accordingly, an absorber with more improved performance can be provided.

In the absorber of either of the above aspects, the upper sheet member may be adhered to the assembly directly or with the lower sheet member interposed therebetween, continuously in a longitudinal direction of the assembly, at least in the vicinity of the left and right edges on the lower surface of the assembly. Therefore, the upper sheet member and assembly are less likely to be separated from each other, which more reliably prevents deformation of the absorber due to twisting.

In the absorber of either of the above aspects, the upper sheet member may be made of an air-through nonwoven fabric. The air-through nonwoven fabric made of a web obtained by adhering staple fibers together by hot air is flexible and has excellent liquid permeability. Accordingly, an absorber can be provided which is flexible, more likely to absorb bodily fluids, and has improved absorbing speeds

The absorber of either of the above aspects further includes a hot melt adhesive layer provided at least in a central region both in a lateral direction and the longitudinal direction of the assembly on the lower surface of the assembly. The hot melt adhesive layer improves the bonding strength between the assembly and upper sheet member or lower sheet member. Therefore, the assembly and upper sheet member or lower sheet member are less likely to be separated from each other even when the absorber is twisted, which prevents deformation of the absorber.

Since the hot melt adhesive layer is provided at least in the central region in the lateral and longitudinal directions of the assembly on the lower surface of the assembly, bodily fluids are repelled to an appropriate degree by the hot melt adhesive layer to diffuse in every direction. This enables efficient and effective use of a larger area of the assembly for absorbing bodily fluids, and improves the absorbing speed, absorbing capacity, and the like.

Since fibers are adhered together by the hot melt adhesive layer at the lower surface of the assembly, the assembly is less likely to be deformed.

In the absorber of either of the above aspects, the hot melt adhesive layer may be provided continuously in the longitudinal direction substantially throughout the entire length of the assembly. Accordingly, bodily fluids are more likely to diffuse in the longitudinal direction substantially throughout the entire length of the assembly, which enables more efficient use of the entire assembly for absorbing bodily fluids.

The hot melt adhesive layer prevents deformation of the absorber due to twisting in the longitudinal direction substantially throughout the entire length of the absorber.

The absorber of either of the above aspects, in which the assembly is a first assembly, may further include a second assembly at least having assembled fibers. The second assembly is provided at the lower surface of the first assembly, and adhered to the first assembly directly or indirectly. An absorber having greater absorbing capacity can be provided by the inclusion of the second assembly.

A disposable absorbent article according to a third aspect of the present invention includes the absorber of either of the above aspects, a liquid-permeable top sheet provided on a skin-facing side of the absorber, and a liquid-impermeable back sheet provided on an exterior side of the absorber. Accordingly, a disposable absorbent article can be provided which prevents deformation of the absorber due to twisting and has improved absorbing speed.

### Brief Description of the Drawings

[fig.1]Fig. 1 is a plan view of a disposable absorbent article including an absorber according to a first embodiment of the present invention, substantially in a flat-out, open state with left and right side joint parts separated.
[fig.2]Fig. 2 is a perspective view of the disposable absorbent article shown in Fig. 1 before separation.
[fig.3]Fig. 3 is a schematic view of a cross-sectional structure of the disposable absorbent article shown in Fig. 1 taken along the line A1-A1.
[fig.4]Fig. 4 is a plan view of the absorber provided in the disposable absorbent article shown in Fig. 1.
[fig.5]Fig. 5 illustrates a variation of the structure of the absorber shown in Fig. 4.
[fig.6]Fig. 6 illustrates a region on a lower surface of an assembly where a hot melt adhesive layer is formed.
[fig.7]Fig. 7 illustrates a region on a lower surface of an assembly where a hot melt adhesive layer is formed.
[fig.8]Fig. 8 illustrates a variation of the structure of the absorber shown in Fig. 5.
[fig.9]Fig. 9 is a schematic view of a cross-sectional structure of the absorber shown in Fig. 8 taken along the line A2-A2.
[fig.10]Fig. 10 is a schematic view of a cross-sectional structure of an absorber according to a second embodiment of the present invention.
[fig.11]Fig. 11 is a schematic view of a cross-sectional structure of an absorber according to a third embodiment of the present invention.
[fig.12]Fig. 12 is a plan view of the absorber shown in Fig. 11 viewed from an upper surface thereof.
[fig.13]Fig. 13 is a cross-sectional view of a variation of the absorber shown in Fig. 11.

### Best Mode for Carrying Out the Invention

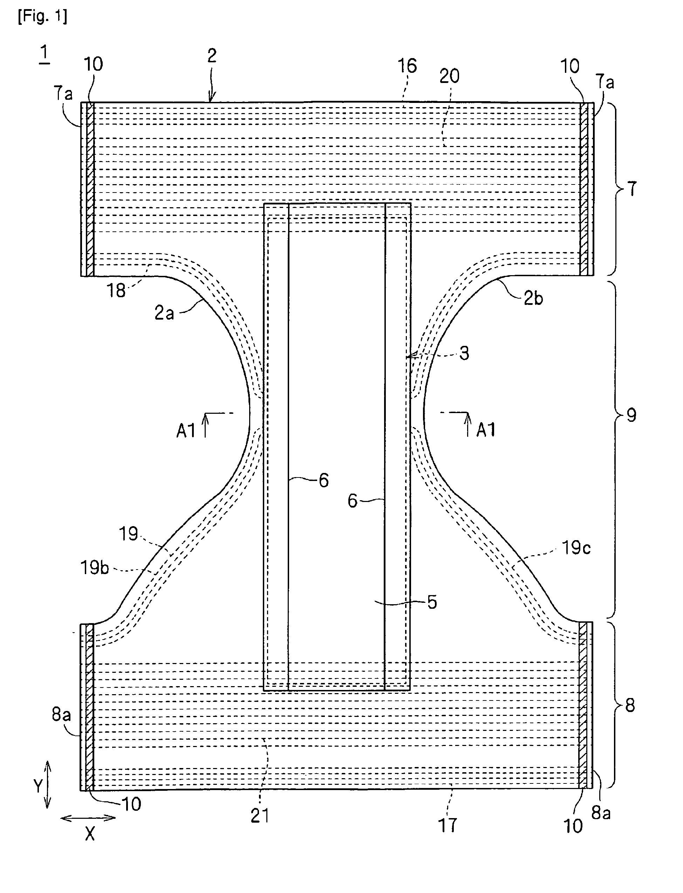

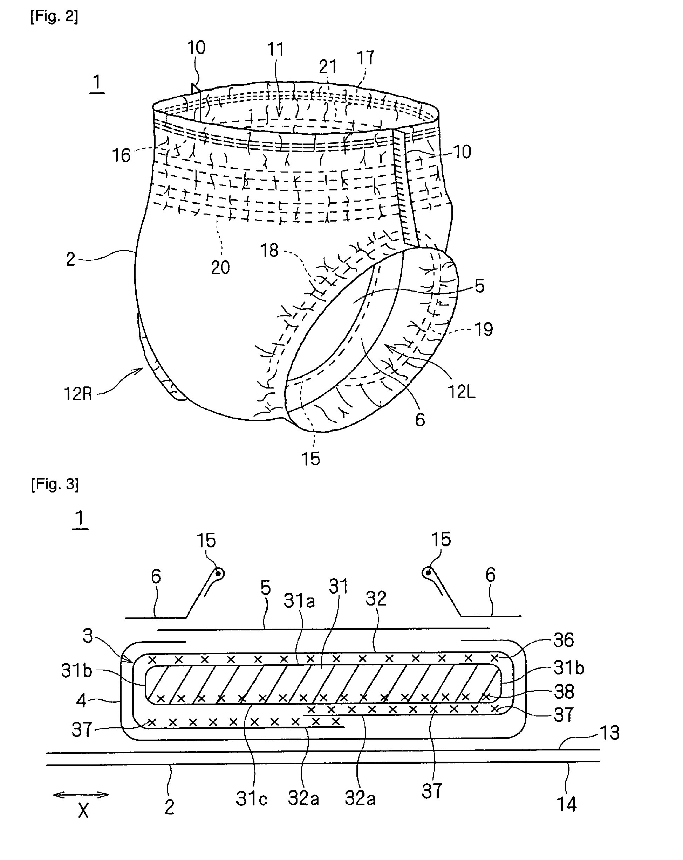

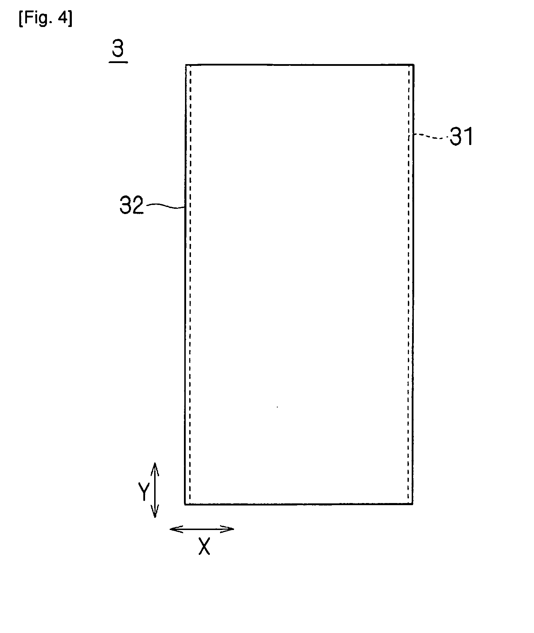

Fig. 1 is a plan view of a disposable absorbent article (hereinafter briefly referred to as "an absorbent article") including an absorber according to a first embodiment of the present invention, substantially in a flat-out, open state with left and right side joint parts separated. Fig. 2 is a perspective view of the disposable absorbent article shown in Fig. 1 before separation. Fig. 3 is a schematic view of a cross-sectional structure of the disposable absorbent article shown in Fig. 1 taken along the line A1-A1. Fig. 4 is a plan view of the absorber provided in the disposable absorbent article shown in Fig. 1. In Figs. 1 to 4 and other drawings which will be described later, the longitudinal direction Y corresponds to the top-to-bottom or front-to-rear direction of a wearer, and the lateral direction X corresponds to the wearer's side-to-side direction.

As shown in Figs. 1 to 4, this absorbent article 1 includes an outer cover 2 provided on the exterior side of the absorbent article 1 and an absorber 3 provided on the skin-facing side of the outer cover 2 for absorbing bodily fluids such as urine of a wearer. The absorbent article 1 also includes a back sheet 4 between the absorber 3 and the outer cover 2 for covering the absorber 3 from the exterior side thereof. The absorbent article 1 further includes a top sheet 5, and right and left side sheets 6 on the skin-facing side of the absorber 3.

The outer cover 2 has a front waist region 7, a rear waist region 8 and a crotch region 9 to be applied to the wearer's front waist region, rear waist region and crotch region, respectively. Herein, the outer cover 2 is of a single-piece design integrally formed of the front waist region 7, crotch region 9 and rear waist region 8, by way of example. Alternatively, the front portion and rear portion having been formed individually may be bonded together to form the outer cover 2.

A left edge 7a of the front waist region 7 and a left edge 8a of the rear waist region 8 of the outer cover 2 are joined to each other by predetermined bonding means while facing each other, so that a left side joint part 10 is formed. A right edge 7b of the front waist region 7 and a right edge 8a of the rear waist region 8 are joined to each other similarly to form a right side joint part 10. Accordingly, the absorbent article 1 is of the garment-type structure having a waist opening 11, and left and right leg openings 12L and 12R. The bonding means for these side joint parts 10 is, for example, one of ultrasonic welding, heating welding, an adhesive (e.g., a hot melt adhesive) and the like, or a combination of some of them.

The outer cover 2 is formed of two sheets 13 and 14. The sheets 13 and 14 are made of, for example, a liquid-repellent nonwoven fabric of synthetic fibers, a synthetic resin film of polyethylene or the like (a liquid-impermeable and breathable film is preferred), or a laminate of these materials. To reduce dampness, a liquid-repellent nonwoven fabric is preferably employed for at least one of the sheets 13 and 14. The nonwoven fabric may include, but limited to, those made of synthetic fibers such as polypropylene, polyethylene, polyester or nylon, or made of composite fibers. The composite fibers may include, but not limited to, a mixture of several types of polyester fibers, a mixture of polyester fibers and polyethylene fibers, or a mixture of polypropylene fibers and polyethylene fibers. The nonwoven fabric is preferably produced by one of spunbonding, air-through method, point bonding, melt blown method and air laid method, or a combination of some of these techniques. Among others, a long staple nonwoven fabric produced by spunbonding or SMS (Spunbonding/Melt blown/Spunbonding) which is a combination of spunbonding and melt blown method is preferable in view of strength. Most preferred is a long staple nonwoven fabric produced by spunbonding.

The absorber 3 is arranged on the skin-facing side of a region of the outer cover 2 that substantially corresponds to the crotch region 9, and bonded to outer cover 2 by bonding means such as a hot melt adhesive with the back sheet 4 interposed therebetween. According to the present embodiment, left and right edges of the back sheet 4 are folded to face the skin-facing side of the absorber 3, as shown in Fig. 3. Left and right edges of the top sheet 5 are overlaid on and bonded to the folded left and right edges of the back sheet 4.

The back sheet 4 is preferably made of a liquid-impermeable material, such as a liquid-repellent nonwoven fabric of synthetic fibers, a synthetic resin film of polyethylene or the like (a liquid-impermeable and breathable film is preferred).

The top sheet 5 is arranged in a central region in the lateral direction X on the skin-facing side of the absorber 3 to extend in the longitudinal direction Y. The left and right side sheets 6 are arranged in the longitudinal direction Y so as to overlap left and right edges of the top sheet 5 from the skin-facing side. A thread-like elastic member 15 is attached in a stretched state to the center-side edge of each of the side sheets 6 in the lateral direction X. Contraction of the elastic members 15 causes part of the side sheets 6 to stand up, so that the side sheets 6 function as standing leg gathers. These standing leg gathers protect against side leakage of bodily fluids.

The top sheet 5 is made of a liquid-permeable nonwoven fabric or woven fabric of synthetic fibers. The nonwoven fabric or woven fabric is preferably made of, but not limited to, hydrophilic fibers such as silk, rayon and pulp, or fibers or composite fibers mentioned as exemplary materials for the sheets 13 and 14 of the outer cover 2. To improve liquid permeability, the nonwoven fabric or woven fabric employed for the top sheet 5 may be treated to be hydrophilic. The side sheets 6 are made of a liquid-repellent nonwoven fabric, for example.

The back sheet 4 and top sheet 5 each have a length in the longitudinal direction Y slightly longer than the length of the absorber 3 in the longitudinal direction Y. Accordingly, the absorber 3 is arranged so as to be sandwiched between the back sheet 4 and top sheet 5.

A waist elastic member 16 is attached in a stretched state to the upper edge of the front waist region 7 of the outer cover 2 to extend along the upper edge. A waist elastic member 17 is attached in a stretched state to the upper edge of the rear waist region 8 of the outer cover 2 to extend along the upper edge.

A front leg elastic member 18 is attached in a stretched state on the front side of the outer cover 2 to extend from one leg-side edge 2a to the other leg-side edge 2b, passing through the central region of the outer cover 2 in the lateral direction X. A rear leg elastic member 19 is attached in a stretched state on the rear side of the outer cover 2 to extend from one leg-side edge 2a to the other leg-side edge 2b, passing through the central region of the outer cover 2 in the lateral direction X. Left and right portions of these leg elastic members 18 and 19 are attached in curves along the inner edges of left and right leg openings 12L and 12R.

These leg elastic members 18 and 19 are attached continuously from one leg-side edge 2a to the other leg-side edge 2b, passing through the central region of the outer cover 2 in the lateral direction X. At least part of the leg elastic members 18 and 19 overlapped by the absorber 3 which will be described later (for example, substantially the entire overlapped section) is then cut short to be weakened. This can avoid disadvantages such as an extremely strong contractile force of the leg elastic members 18 and 19 causing an uncomfortable fit, and contraction of the leg elastic members 18 and 19 causing twisting of the absorber 3.

A body elastic member 20 is attached in a stretched state in the lateral direction X to a region of the front waist region 7 of the outer cover 2 to be positioned at the wearer's waist (a middle region of the front waist region 7 in the longitudinal direction Y). A body elastic member 21 is attached in a stretched state in the lateral direction X to a region of the rear waist region 8 to be positioned at the wearer's waist (a middle region of the rear waist region 8 in the longitudinal direction Y).

These elastic members 16 to 21 are made of one or more (e.g., a plurality of) elastic materials (e.g., elastic threads). For the elastic materials, elastic stretchable materials (polyurethane threads, natural rubber, or the like) widely employed for disposable absorbent articles are generally suitable. Strip polyurethane films may be employed as the elastic materials. The elastic members 16 to 21 are sandwiched between the two sheets 13 and 14 constituting the outer cover 2. Bonding of the sheets 13 and 14 to each other and bonding of the elastic members 16 to 21 to the sheet members 13 and 14 are achieved by using bonding means such as an adhesive (e.g., hot melt adhesive), heating welding, ultrasonic welding or the like. For example, as the elastic members 16 to 21, polyurethane threads of 300 to 2000 decitex are stretched 1.1 to 5.0 times their original length to be sandwiched between the sheets 13 and 14.

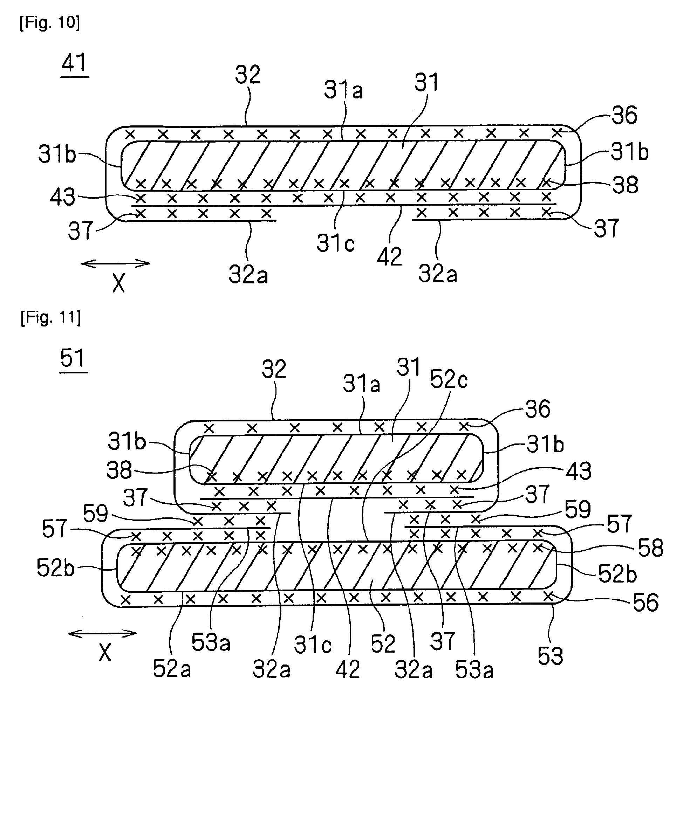

The structure of the absorber 3 will now be described. The absorber 3 includes an assembly 31 at least having assembled fibers, and a sheet member 32 for covering the assembly 31. The sheet member 32 corresponds to an upper sheet member according to the present invention. In the present embodiment, the absorber 3 is arranged with its upper surface facing the wearer's skin. Alternatively, the absorber 3 may be arranged with its upper surface facing the exterior side. This also applies to absorbers 41, 51 and 71 shown in Figs. 10 to 13 which will be described later.

The assembly 31 is mainly formed of assembled fibers. For the fibers, fibrillated pulp fibers or cellulosic fibers are mainly employed, into which thermally-welded fibers or the like may be mixed according to necessity. The assembly 31 preferably contains super absorbent polymer powder. Supposing that the fibers and super absorbent polymer powder constitute 100 % by mass in total, the super absorbent polymer powder is preferably contained by not less than 20 % by mass. The super absorbent polymer powder can ensure excellent absorbing performance, and retains bodily fluids therein, to thereby prevent back flow of the bodily fluids. However, mixing an extremely large amount of super absorbent polymer powder will stiffen the assembly 31. Accordingly, the super absorbent polymer powder is preferably mixed by not more than 80 % by mass, and more preferably, by not more than 60 % by mass. For the super absorbent polymer powder, polyacrylate-based one or the like commonly used in this field is preferable.

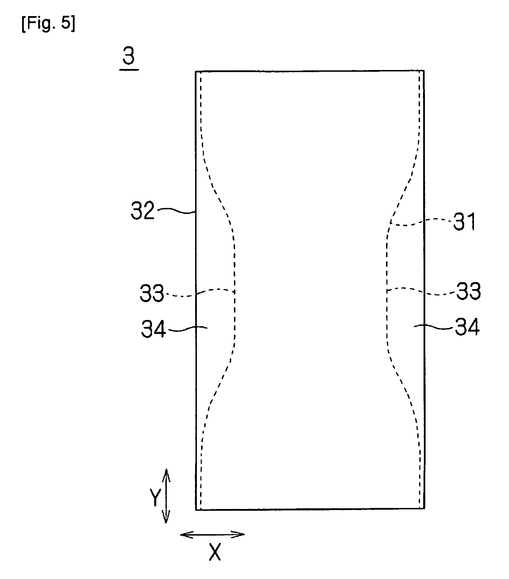

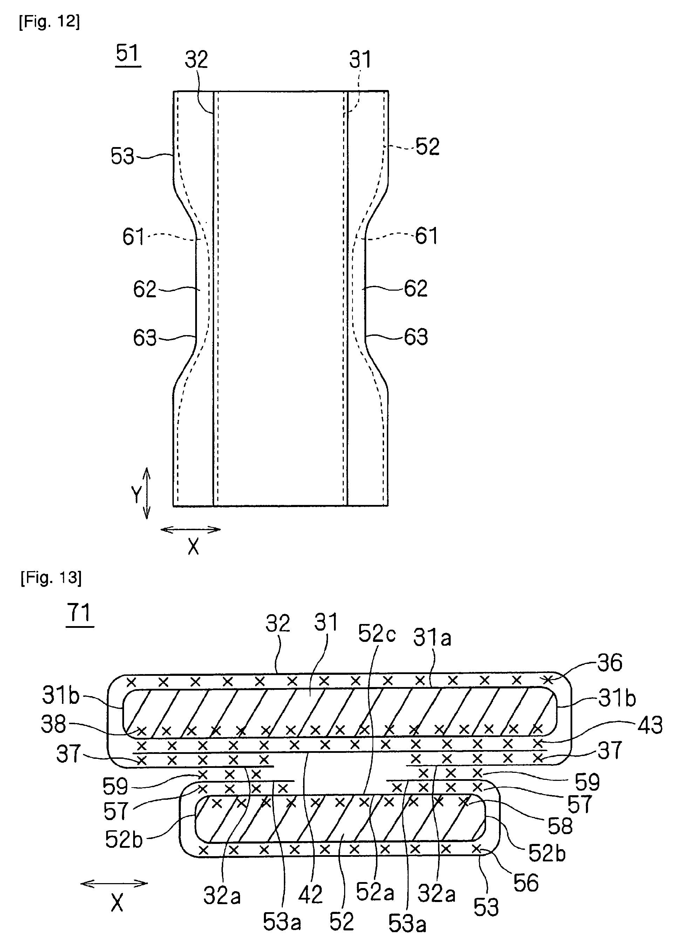

The assembly 31 may take various forms of plan shape including, but not limited to, sandglass, substantially rectangle and substantially oval. In the present embodiment, the assembly 31 has a rectangular plan shape, as shown in Fig. 4. As an alternative, the assembly 31 may have a sandglass plan shape as shown in Fig. 5, for example, having recessed portions 33 in the central region of the assembly 31 in the longitudinal direction Y (corresponding to the wearer's crotch region). In the structure shown in Fig. 5, the sheet member 32 has its left and right edges folded by constant width in the longitudinal direction Y to face the lower surface 31c of the assembly 31. Accordingly, crotch flaps 34 where the assembly 31 is absent are formed in regions of the sheet member 32 that correspond to the recessed portions 33 of the assembly 31. In this case, part of the crotch flaps 34 of the sheet member 32 may be cut away, as shown in Fig. 12 which will be described later.

The sheet member 32, made of a liquid-permeable nonwoven fabric, covers the upper surface 31a, left and right edges 31b of the assembly 31, and also covers regions at least in the vicinity of the edges 31b of the assembly 31 on the lower surface 31c. The sheet member 32 is adhered to the assembly 31 continuously in the longitudinal direction Y in the regions at least in the vicinity of the edges 31b on the lower surface 31c of the assembly 31.

Covering the assembly 31 with the sheet member 32 as described above offers the following effects. First, the sheet member 32 covering the assembly 31 in the above-described manner is made of a nonwoven fabric having high liquid-permeability, resisting tearing, and having excellent flexibility. Accordingly, the absorber 3 resists tearing and has flexibility. Also, the absorbing speed of the absorber 3 is improved, so that bodily fluids such as urine pass immediately through the sheet member 32 to reach the assembly 31 to be absorbed therein.

Moreover, since the sheet member 32 covering the assembly 31 in the above-described manner is adhered to the assembly 31 in the regions at least in the vicinity of the edges 31b on the lower surface 31c of the assembly 31, the assembly 31 and sheet member 32 are less likely to be separated from each other even when the absorber 3 is twisted, which prevents deformation of the absorber 3. Further because the sheet member 32 is adhered to the assembly 31 continuously in the longitudinal direction Y in the regions at least in the vicinity of the edges 31b on the lower surface 31c of the assembly 31, the assembly 31 and sheet member 32 are even less likely to be separated from each other, which more reliably prevents deformation of the absorber 3 due to twisting.

In the present embodiment, the sheet member 32 is arranged to cover the entire circumference of the assembly 31. Accordingly, the left and right edges 32a of the sheet member 32 folded to face the lower surface 31c of the assembly 31 are adhered to overlap each other on the lower surface 31c of the assembly 31. The entire circumference of the assembly 31 is thereby covered by the single sheet member 32, which, for example, can simplify the structure and manufacturing process of the absorber 3.

In the present embodiment, an adhesive (e.g., hot melt adhesive) 36 is applied to regions of the sheet member 32 that face the upper surface 31a of the assembly 31 to adhere the sheet member 32 to the upper surface 31a of the assembly 31. An adhesive (e.g., hot melt adhesive) 37 is applied to regions of the sheet member 32 that face the lower surface 31c of the assembly 31 to adhere the sheet member 32 to the lower surface 31c of the assembly 31. The adhesive 37 also adheres the left and right edges of the sheet member 32 to each other.

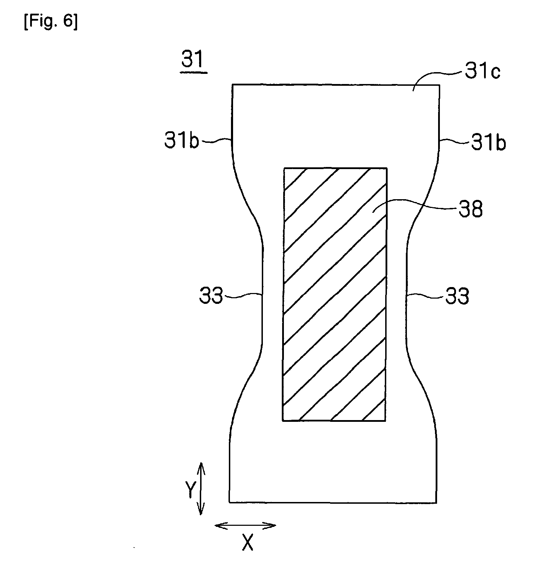

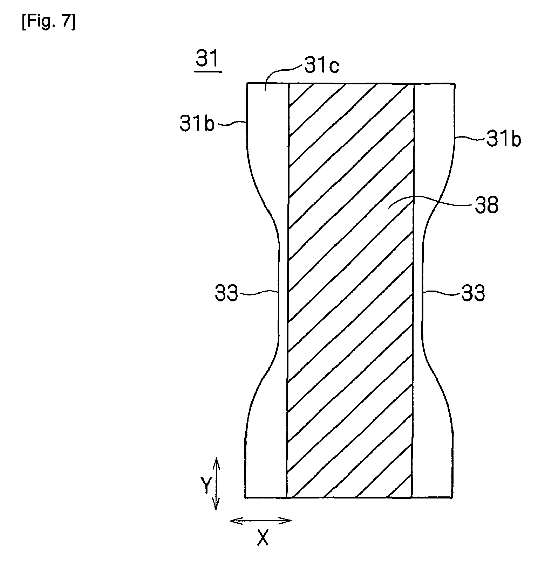

A hot melt adhesive layer 38 is formed at the lower surface 31c of the assembly 31. The structure of the hot melt adhesive layer 38 will specifically be described supposing that the assembly 31 has a substantially sandglass plan shape. As shown in Fig. 6, the hot melt adhesive layer 38 is formed at least in the central region of the assembly 31 (hatched region in Fig. 6) in the lateral and longitudinal directions X and Y. More preferably, as shown in Fig. 7, the hot melt adhesive layer 38, having a width slightly narrower than the width between the recessed portions 33 of the assembly 31 in the lateral direction X, is provided on the lower surface 31c of the assembly 31 continuously in the longitudinal direction Y throughout substantially the entire length of the assembly 31. In the case where the assembly 31 has a rectangular plan shape, the hot melt adhesive layer 38 may be formed on substantially the entire region of the lower surface 31c of the assembly 31.

The hot melt adhesive layer 38 is formed by means of a contact-type or noncontact-type application apparatus. For example, the hot melt adhesive layer 38 is formed by applying a hot melt adhesive onto the lower surface 31c of the assembly 31 by a curtain coating apparatus. The curtain coating apparatus has a plurality of discharge outlets arranged in a line for discharging a hot melt adhesive, from which the hot melt adhesive is discharged in the form of curtain. As a variation of curtain coating, the hot melt adhesive may be applied in spiral pattern, zigzag pattern, or substantially fiber pattern by air. For example, a melt blown coater is employed for applying a hot melt adhesive in substantially fiber pattern.

Such hot melt adhesive layer 38 achieves the following effects. First, since the hot melt adhesive layer 38 improves the bonding strength between the assembly 31 and sheet member 32, the assembly 31 and sheet member 32 are less likely to be separated from each other even when the absorber 3 is twisted, which more reliably prevents deformation of the absorber 3.

Second, since the hot melt adhesive layer 38 is provided at least in the central region of the assembly 31 both in the lateral and longitudinal directions X and Y on the lower surface 31c of the assembly 31, bodily fluids are repelled to an appropriate degree by the hot melt adhesive layer 38 to diffuse in every direction. This enables effective and efficient use of a larger area of the assembly 31 for absorbing bodily fluids, which improves the absorbing speed and absorbing capacity.

Further, the assembly 31 is less likely to be deformed because fibers are adhered together by the hot melt adhesive layer 38 at the lower surface 31c of the assembly 31.

Furthermore, when the hot melt adhesive layer 38 is provided continuously in the longitudinal direction Y substantially throughout the entire length of the assembly 31, bodily fluids are more likely to diffuse in the longitudinal direction Y substantially along the entire length of the assembly 31. This enables more efficient use of the entire assembly 31 for absorbing bodily fluids. Moreover, in this case, the hot melt adhesive layer 38 prevents deformation of the absorber 3 due to twisting, substantially along the entire length in the longitudinal direction Y.

Herein, the hot melt adhesive employed in the present embodiment includes, but not limited to, (a) a styrene hot melt adhesive such as SIS (styrene-isoprene block copolymer) or SBS (styrene-butadiene block copolymer) containing a styrene block polymer as a base polymer, (b) amorphous polyalphaolefin (APAO) such as ethylenepropylene copolymer, (c) an olefin hot melt adhesive containing commonly-used olefin as a base polymer, or (d) ethylene-vinyl acetate copolymer (EVA), polyester or an adhesive containing ethylene-vinyl acetate copolymer as a base polymer. A styrene hot melt adhesive is particularly preferable.

As the nonwoven fabric employed for the sheet member 32, an air-through nonwoven fabric produced by adhering staple fibers together by hot air is preferable in view of flexibility and liquid-permeability. A more preferred air-through nonwoven fabric is an air-through nonwoven fabric made of synthetic fibers. One made of 100% polyethylene terephthalate (PET) is particularly preferable. A still more preferred nonwoven fabric contains large-diameter staple fibers of not less than 10 denier and not more than 20 denier having a length of not less than 50 mm and not more than 70 mm by about 50 to 70%, and small-diameter staple fibers of not less than 1 denier and less than 10 denier having a length of not less than 10 mm and not more than 60 mm by about 30 to 50%. The small-diameter staple fibers may have a core-sheath construction in which the sheath has a melting temperature slightly lower than that of the core, in order to improve the adhesion properties by hot air. Accordingly, the absorber 3 and absorbent article 1 can be provided which are flexible, more likely to absorb bodily fluids, and have high absorbing speed. It should be noted that an air-through nonwoven fabric includes nonwoven fabrics produced by the air-through method.

As described above, the use of the absorber 3 according to the present embodiment can provide the absorbent article 1 which prevents deformation of the absorber 3 due to twisting and has improved absorbing speed.

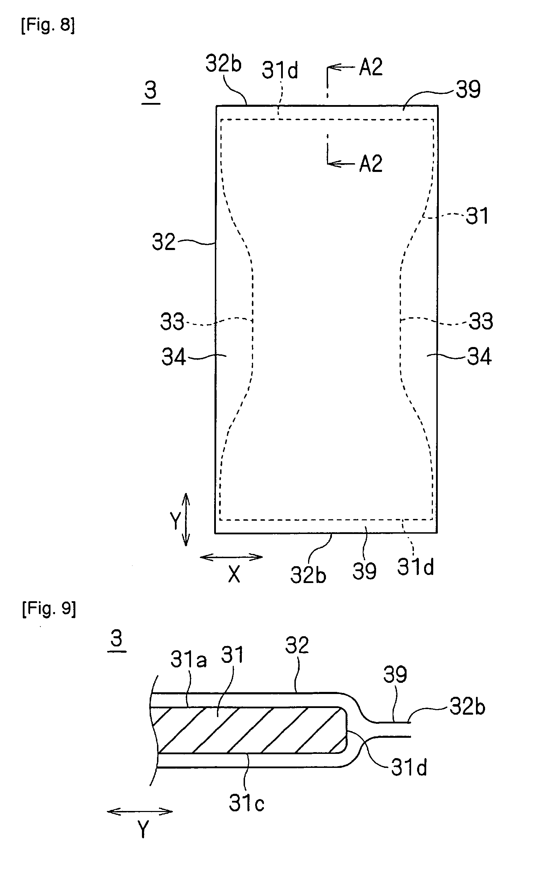

Fig. 8 illustrates a variation of the structure of the absorber 3 shown in Fig. 5 mentioned above. Fig. 9 is a schematic view of a cross-sectional structure of the absorber 3 shown in Fig. 8 taken along the line A2-A2. In the structure shown in Figs. 4 and 5 mentioned above, front and rear ends 32b of the sheet member 32 (in the longitudinal direction Y) are aligned substantially with the front and rear ends 31d of the assembly 31, and are left open. In this regard, the structure shown in Figs. 8 and 9 may alternatively be employed. More specifically, in the structure shown in Figs. 8 and 9, the front and rear ends 32b of the sheet member 32 (see Figs. 8 and 9) project outwards from the front and rear ends 31d of the assembly 31, respectively. The ends 32b of the sheet member 32 are adhered to each other on the surfaces respectively facing the upper surface 31a and lower surface 31c of the assembly 31, and are closed. Accordingly, the front and rear ends 32b of the sheet member 32 have flaps 39 where the assembly 31 is absent. Fibers and other elements constituting the assembly 31 can thereby be enclosed within the sheet member 32.

Fig. 10 is a schematic view of a cross-sectional structure of an absorber according to a second embodiment of the present invention. An absorber 41 according to the second embodiment substantially differs from the absorber 3 according to the above-described first embodiment only in that a lower sheet member 42 and its relative parts are added to the sheet member 32 provided as an upper sheet member 32. Corresponding parts are denoted by like reference numerals, and description thereof will not be repeated. The absorber 41 according to the second embodiment is provided in the absorbent article 1 or the like substantially similarly to the absorber 3 according to the first embodiment.

In the absorber 41 according to the present embodiment, the lower sheet member 42 is adhered to the lower surface 31c of the assembly 31, as shown in Fig. 10. The length of the lower sheet member 42 in the longitudinal direction Y is, for example, aligned with that of the upper sheet member 32. The lower sheet member 42 has a width in the lateral direction X substantially equal or slightly narrower than the width of the assembly 31 in the lateral direction X. An adhesive (e.g., hot melt adhesive) 43 is applied to a surface of the lower sheet member 42 that faces the assembly 31. The lower sheet member 42 and assembly 31 are adhered to each other by the adhesive 43 and the aforementioned hot melt adhesive layer 38.

In the present embodiment, the left and right edges 32a of the upper sheet member 32 folded to face the lower surface 31c of the assembly 31 are respectively overlaid on the left and right edges of the lower sheet 42 from the side of the lower surface (the exterior side) to be adhered to the lower sheet member 42. More specifically, the left and right edges 32a of the upper sheet member 32 are adhered to the assembly 31 with the lower sheet member 42 interposed therebetween so as to cover regions at least in the vicinity of the left and right edges 31b on the lower surface 31c of the assembly 31. In the structure of the present embodiment, the left and right edges 32a of the upper sheet member 32, spaced from each other in the lateral direction X without overlapping each other, are adhered to the lower sheet member 42 as shown in the structure in Fig. 3. The upper sheet member 32 and lower sheet member 42 are adhered to each other by, for example, an adhesive (e.g., hot melt adhesive) 37 applied to a region of the upper sheet member 32 that faces the lower sheet member 42.

The lower sheet member 42 may be made of a material similar to that of the upper sheet member 32, or another material may be selected. For example, in order to improve the strength of the absorber 3, the lower sheet member 42 may be made of a nonwoven fabric having a higher strength than the upper sheet member 32. For more efficient use of the entire assembly 31 for absorbing bodily fluids such as urine, the lower sheet member 42 may be made of a crepe paper.

With the above-described structure, the present embodiment achieves substantially similar effects to those of the aforementioned first embodiment. Particularly in the present embodiment, materials for the upper sheet member 32 and lower sheet member 42 can be selected individually. Accordingly, the absorber 3 and absorbent article 1 can be provided which are more superior in strength (e.g., less likely to tear), absorbing performance, and the like.

Fig. 11 is a schematic view of a cross-sectional structure of an absorber according to a third embodiment of the present invention. Fig. 12 is a plan view of the absorber shown in Fig. 11 viewed from an upper surface thereof. An absorber 51 according to the third embodiment is also provided in the disposable absorbent article 1 or the like substantially similarly to the absorbers 3 and 41 according to the first and second embodiments.

In the absorber 51 according to the present embodiment, as shown in Figs. 11 and 12, another assembly 52 is added under the absorber 41 which is similar to the second embodiment but narrower in the lateral direction X. In the present embodiment, the upper assembly 31 will be referred to as first assembly 31, and the lower assembly 52 will be referred to as second assembly 52.

The second assembly 52 is covered by a sheet member 53 of a material similar to that of the upper sheet member 32. The sheet member 53 is attached so as to enclose the second assembly 52 from the side of the lower surface for covering a lower surface 52a, and left and right edges 52b of the second assembly 52. Left and right edges 53a of the sheet member 53 are folded to face an upper surface 52c of the second assembly 52. The left and right edges 53a of the sheet member 53, spaced from each other in the lateral direction X without overlapping each other, are adhered to the upper surface 52c of the second assembly 52. Accordingly, the sheet members 32 and 53 are absent between the first assembly 31 and second assembly 52 in the central part of the absorber 51 in the lateral direction X, and only the sheet member 42 is present. This allows bodily fluids such as urine outflowing into the first assembly 31 to pass immediately therethrough toward the second assembly 52.

An adhesive (e.g., hot melt adhesive) 56 is applied to a region of the sheet member 53 that faces the lower surface 52a of the second assembly 52 to adhere the sheet member 53 to the lower surface 52a of the second assembly 52. An adhesive (e.g., hot melt adhesive) 57 is applied to regions of the sheet member 53 that face the upper surface 52c of the second assembly 52 to adhere the sheet member 53 to the upper surface 52c of the second assembly 52.

A hot melt adhesive layer 58 is formed at the upper surface 52c of the second assembly 52. This hot melt adhesive layer 58 is formed substantially similarly to the aforementioned hot melt adhesive layer 38. This improves the bonding strength between the second assembly 52 and sheet member 53, and prevents the second assembly 52 from being deformed. Also, bodily fluids reached the second assembly 52 are repelled to an appropriate degree by the hot melt adhesive layer 58 to diffuse in every direction. This enables effective and efficient use of a larger area of the second assembly 52 for absorbing bodily fluids, which improves the absorbing speed and absorbing capacity.

The elements of the first assembly 31 and those of the second assembly 52 are adhered by, for example, the hot melt adhesive layer 58 formed at the upper surface 52c of the second assembly 52 and an adhesive (e.g., hot melt adhesive) 59 applied between opposing regions of the sheet members 32 and 53. It should be noted that the adhesive 59 may be omitted when the hot melt adhesive layer 58 alone ensures a sufficient bonding strength between the first assembly 31 and second assembly 52.

In the present embodiment, the second assembly 52 has a width in the lateral direction X larger than the width of the first assembly 31 in the lateral direction X. The second assembly 52 is of a sandglass plan shape having recessed portions 61 in a central region in the longitudinal direction Y (corresponding to the wearer's crotch region). Accordingly, crotch flaps 62 where the second assembly 52 is absent are formed in regions of the sheet member 53 that correspond to the recessed portions 61 of the second assembly 52. Further, in the structure shown in Fig. 12, part of the crotch flaps 62 of the sheet member 53 is cut away to have recessed portions, so that trims 63 are formed.

As described above, the present embodiment not only achieves the effects obtained by the absorber 3 according to the second embodiment, but also can provide the absorber 51 and absorbent article 1 which have greater absorbing capacity by the inclusion of the second assembly 52.

Fig. 13 is a sectional view of a variation of the absorber according to the present embodiment. In an absorber 71 according to this variation, the first assembly 31 has a width in the lateral direction X larger than the width of the second assembly 52 in the lateral direction X, as shown in Fig. 13. As another variation, the first assembly 31 may have a width in the lateral direction X substantially equal to the width of the second assembly 52 in the lateral direction X.

In the above-described embodiments, the absorbers 3, 41, 51 and 71 are applied to the garment-type absorbent article 1. However, the absorbers 3, 41, 51 and 71 may be applied to a hook-and-loop-type disposable diaper or a disposable absorbent article for combined use as a garment and a hook-and-loop-type diaper. Such a disposable absorbent article for combined use represents an article having breaking parts for separating the waist sections and fastening members for re-fastening after separation, which is of the garment-type structure when the breaking parts are not separated, and of the hook-and-loop-type structure when the breaking parts are separated.

The absorbers 3, 41, 51 and 71 according to the above-described embodiments may also be applied to various other types of disposable absorbent articles including, but not limited to, incontinence pads, sanitary napkins and sanitary garments.

## Claims

1. An absorber provided in a disposable absorbent article, comprising:
an assembly (31) at least including assembled fibers;
an upper sheet member (32) made of a liquid-permeable nonwoven fabric, covering an upper surface and left and right edges of said assembly (31), and covering at least said left and right edges on a lower surface of said assembly (31),
an adhesive (36) applied to regions of the sheet member (32) that face the upper surface of the assembly (31), and
a hot melt adhesive layer (38) provided at least in a central region both in a lateral direction and a longitudinal direction of said assembly (31) on said lower surface of said assembly (31), wherein
said upper sheet member (32) is adhered to said assembly (31) in at least said left and right edges on said lower surface of said assembly (31).

2. The absorber according to claim 1, wherein
said upper sheet member (32) is adhered directly to said assembly (31) continuously in the longitudinal direction of said assembly (31), in at least said left and right edges on said lower surface of said assembly (31).

3. The absorber according to claim 1, further comprising a lower sheet member (42) adhered to said lower surface of said assembly (31), wherein
said upper sheet member (32) covering said upper surface and said left and right edges of said assembly (31), and covering, from a side of said lower surface of said lower sheet member (42), at least said left and right edges on said lower surface of said assembly (31),
said upper sheet member (32) is adhered to said assembly (31) with said lower sheet member (42) interposed therebetween, in at least said left and right edges on said lower surface of said assembly (31).

4. The absorber according to claim 3, wherein
said upper sheet member (32) is adhered to said assembly (31) with said lower sheet member (42) interposed therebetween, continuously in the longitudinal direction of said assembly (31), in at least said left and right edges on said lower surface of said assembly (31).

5. The absorber according to any one of claims 1 to 4, wherein said upper sheet member (32) is made of an air-through nonwoven fabric.

6. The absorber according to any one of claims 1 to 5, wherein
said hot melt adhesive layer (38) is provided continuously in said longitudinal direction throughout an entire length of said assembly (31).

7. The absorber according to any one of claims 1 to 6, wherein
said assembly (31) is a first assembly,
said absorber (3, 51) further comprising a second assembly (52) at least including assembled fibers, provided at said lower surface of said first assembly (31) and adhered to said first assembly (31) directly or indirectly.

8. A disposable absorbent article comprising:
said absorber (3, 41, 51) according to any one of claims 1 to 7;
a liquid-permeable top sheet (5) provided on a skin-facing side of said absorber (3, 41, 51); and
a liquid-impermeable back sheet (4) provided on an exterior side of said absorber (3, 41,51).

## Patentansprüche

1. Absorber, der in einem saugfähigen Einwegartikel vorgesehen ist, umfassend:
eine Anordnung (31), die zumindest angeordnete bzw. zusammengesetzte Fasern enthält;
ein oberes Folien- bzw. Lagenglied (32), das aus einem flüssigkeitsdurchlässigen Vliesstoff bzw. Faserstoff besteht, das eine obere Fläche bzw. Oberfläche und linke und rechte Kanten bzw. Ränder der Anordnung (31) abdeckt und das zumindest die linken und rechten Kanten bzw. Ränder an einer unteren Fläche bzw. Oberfläche der Anordnung (31) abdeckt,
ein Haft- bzw. Klebemittel (36), das an Bereichen des Folien- bzw. Lagenglieds (32) angebracht ist, die der oberen Fläche bzw. Oberfläche der Anordnung (31) zugewandt sind, und
eine Heißschmelzhaft- bzw. -klebemittelschicht (38), die zumindest in einem zentralen Bereich in einer lateralen Richtung und einer longitudinalen Richtung der Anordnung (31) an der unteren Fläche bzw. Oberfläche der Anordnung (31) vorgesehen ist, wobei
das obere Folien- bzw. Lagenglied (32) an der Anordnung (31) in zumindest den linken und rechten Kanten bzw. Rändern der unteren Fläche bzw. Oberfläche der Anordnung (31) angehaftet bzw. angeklebt ist.

2. Absorber nach Anspruch 1, wobei
das obere Lagenglied (32) direkt an der Anordnung (31) kontinuierlich bzw. durchgängig in der longitudinalen Richtung der Anordnung (31) in zumindest den linken und rechten Kanten der unteren Fläche der Anordnung (31) angehaftet ist.

3. Absorber nach Anspruch 1, ferner umfassend ein unteres Folien- bzw. Lagenglied (42), das an die untere Fläche der Anordnung (31) angehaftet ist, wobei
das obere Lagenglied (32) die obere Fläche und die linken und rechten Kanten der Anordnung (31) abdeckt und von einer Seite der unteren Fläche des unteren Lagenglieds (42) aus zumindest die linken und rechten Kanten an der unteren Fläche der Anordnung (31) abdeckt,
das obere Lagenglied (32) an der Anordnung (31) mit dem unteren Lagenglied (42) dazwischen angeordnet angehaftet ist, und zwar in zumindest den linken und rechten Kanten an der unteren Fläche der Anordnung (31).

4. Absorber nach Anspruch 3, wobei
das obere Lagenglied (32) an der Anordnung (31) mit dem unteren Lagenglied (42) dazwischen angeordnet angehaftet ist, und zwar kontinuierlich bzw. durchgängig in der longitudinalen Richtung der Anordnung (31) in zumindest den linken und rechten Kanten an der untere Fläche der Anordnung (31).

5. Absorber nach einem der Ansprüche 1 bis 4, wobei das obere Lagenglied (32) aus einem luftdurchlässigen Faserstoff bzw. Vliesstoff besteht.

6. Absorber nach einem der Ansprüche 1 bis 5, wobei
die Heißschmelzhaftmittelschicht (38) durchgängig in der longitudinalen Richtung über die gesamte Länge der Anordnung (31) vorgesehen ist.

7. Absorber nach einem der Ansprüche 1 bis 6, wobei
die Anordnung (31) eine erste Anordnung ist,
der Absorber (3, 51) ferner eine zweite Anordnung (52) umfasst, die zumindest angeordnete bzw. zusammengesetzte Fasern umfasst, vorgesehen an der unteren Fläche der ersten Anordnung (31) und direkt oder indirekt an die erste Anordnung (31) angehaftet.

8. Saugfähiger Einwegartikel, umfassend:
den Absorber (3, 41, 51) nach einem der Ansprüche 1 bis 7;
eine flüssigkeitsdurchlässige oberste bzw. Deckfolie bzw. -lage (5), die auf einer der Haut zugewandten Seite des Absorbers (3, 41, 51) vorgesehen ist; und
eine flüssigkeitsundurchlässige hinterste bzw. Rückenfolie bzw. -lage (4), die auf einer Außenseite des Absorbers (3, 41, 51) vorgesehen ist.

## Revendications

1. Elément absorbant disposé dans un élément absorbant jetable, comprenant:
un ensemble (31) incluant au moins des fibres assemblées ;
un élément de feuille supérieure (32) réalisé en matériau non tissé perméable aux liquides, couvrant une surface supérieure et des bords gauche et droite dudit ensemble (31), et couvrant au moins lesdits bords gauche et droite sur une surface intérieure dudit ensemble (31),
un adhésif (36) appliqué à des régions de l'élément de feuille supérieure (32) qui font face à la surface supérieure de l'ensemble (31), et
une couche d'adhésif thermo-fusible (38) prévue au moins dans une région centrale à la fois dans une direction latérale et une direction longitudinale dudit ensemble (31) sur ladite surface inférieure dudit ensemble (31), dans lequel
ledit élément de feuille supérieure (32) vient adhérer sur ledit ensemble (31) dans au moins lesdits bords gauche et droite sur ladite surface inférieure dudit ensemble (31).

2. Elément absorbant selon la revendication 1, dans lequel
ledit élément de feuille supérieure (32) vient adhérer directement sur ledit ensemble (31) de façon continue dans la direction longitudinale dudit ensemble (31), dans au moins lesdits bords gauche et droite sur ladite surface inférieure dudit ensemble (31).

3. Elément absorbant selon la revendication 1, comprenant également un élément de feuille inférieure (42) qui vient adhérer sur ladite surface inférieure dudit ensemble (31), dans lequel
ledit élément de feuille supérieure (32) couvrant ladite surface supérieure et lesdits bords gauche et droite dudit ensemble (31), et couvrant, à partir d'un côté de ladite surface inférieure dudit élément de feuille inférieure (42), au moins lesdits bords gauche et droite sur ladite surface inférieure dudit ensemble (31),
ledit élément de feuille supérieure (32) vient adhérer sur ledit ensemble (31) avec interposition dudit élément de feuille inférieure (42), dans au moins lesdits bords gauche et droite sur ladite surface inférieure dudit ensemble (31).

4. Elément absorbant selon la revendication 3, dans lequel
ledit élément de feuille supérieure (32) vient adhérer sur ledit ensemble (31) avec interposition dudit élément de feuille inférieure (42), de façon continue dans la direction longitudinale dudit ensemble (31), dans au moins lesdits bords gauche et droite sur ladite surface inférieure dudit ensemble (31).

5. Elément absorbant selon l'une quelconque des revendications 1 à 4, dans lequel ledit élément de feuille supérieure (32) est réalisé en matériau non tissé perméable à l'air.

6. Elément absorbant selon l'une quelconque des revendications 1 à 5, dans lequel ladite couche d'adhésif thermo-fusible (38) est prévue de façon continue dans ladite direction longitudinale à travers toute une longueur dudit ensemble (31).

7. Elément absorbant selon l'une quelconque des revendications 1 à 6, dans lequel ledit ensemble (31) est un premier ensemble,
ledit élément absorbant (3, 51) comprenant également un deuxième ensemble (52) comprenant au moins des fibres assemblées, prévu sur ladite surface inférieure dudit premier ensemble (31) et venant adhérer sur ledit premier ensemble (31) directement ou indirectement.

8. Elément absorbant jetable, comprenant:
ledit élément absorbant (3, 41, 51) selon l'une quelconque des revendications 1 à 7 ;
une feuille supérieure (5) perméable aux liquides prévue sur un côté tourné vers la peau dudit élément absorbant (3, 41, 51) ; et
une feuille arrière (4) imperméable aux liquides prévue sur un côté extérieur dudit élément absorbant (3, 41, 51).
